# DEMANDE DE BREVET EUROPEEN

(11) **EP 1 317 920 A1**
(43) Date de publication de la demande: **11.06.2003**
(21) Numéro de dépôt: 02292836.0
(22) Date de dépôt: 14.11.2002
(51) Int. Cl.: A61K 7/48, A61K 7/42

(54) **Composition autobronzante contenant un dérivé du 2-hydroxybenzophénone aminosubstitué et un agent autobronzant**

(30) Priorité: 07.12.2001 FR 0115856
(71) Demandeur: L'OREAL, 75008 Paris (FR)
(72) Inventeur: Candau, Didier, 91570 Bievres (FR)
(74) Mandataire: Miszputen, Laurent

(57) **Abrégé**

La présente invention se rapporte à une composition cosmétique et/ou dermatologique plus particulièrement destinée au bronzage et/ou au brunissage artificiels de la peau et comprenant, dans un support cosmétiquement acceptable, au moins un dérivé de 2-hydroxybenzophénone aminosubstitué particulier et au moins un agent autobronzant.

La présente invention se rapporte encore à un procédé de traitement cosmétique pour bronzer ou brunir artificiellement la peau et à l'utilisation d'au moins un dérivé de 2-hydroxybenzophénone aminosubstitué particulier pour améliorer la coloration et/ou la stabilité d'un agent autobronzant.

L'invention concerne également les applications de ces compositions à la coloration de la peau proche du bronzage naturel de la peau.

## Description

La présente invention se rapporte à une composition cosmétique et/ou dermatologique plus particulièrement destinée au bronzage et/ou au brunissage artificiel de la peau et comprenant, dans un support cosmétiquement acceptable, au moins un dérivé de 2-hydroxybenzophénone aminosubstitué particulier et au moins un agent autobronzant.

La présente invention se rapporte encore à un procédé de traitement cosmétique pour bronzer ou brunir artificiellement la peau et à l'utilisation d'au moins un dérivé de 2-hydroxybenzophénone aminosubstitué particulier pour améliorer la coloration et/ou la stabilité d'un agent autobronzant.

L'invention concerne également les applications de ces compositions à la coloration de la peau proche du bronzage naturel de la peau.

"Par agent autobronzant" au sens de la présente demande, on entend un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire des réactions voire une altération de celle-ci, notamment dans le cas d'une peau sensible ou d'une peau continuellement exposée au rayonnement solaire : érythème, brûlure, perte d'élasticité, apparition de rides, vieillissement prématuré.

Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés, parmi ceux-ci on compte des composés mono- ou polycarbonylés tels que par exemple l'isatine, l'alloxane, la ninhydrine, le glycéraldéhyde, l'aldéhyde mésotartrique, la glutaraldéhyde, l'érythrulose, la dihydroxyacétone (DHA).

La DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. L'intensité de la coloration obtenue sur la peau et /ou sa tenue au cours du temps (résistance au lavage) et/ou la rapidité avec laquelle la coloration se développe, sont souvent jugées insuffisantes par les utilisateurs de compositions autobronzantes à base de DHA.

Un autre problème posé par les compositions à base de DHA est qu'elles présentent la fâcheuse tendance, plus ou moins prononcée selon la nature du milieu dans lequel elles sont formulées, à se dégrader au cours du temps. Ces problèmes liés au stockage et/ou à la conservation des compositions à base de DHA se traduisent généralement à terme par un jaunissement non souhaitable de ces compositions.

Il existe donc une demande croissante de produits autobronzants agissant rapidement et conférant une coloration proche du bronzage naturel.

De manière surprenante et avantageuse, la demanderesse a trouvé que l'utilisation d'un dérivé de 2-hydroxybenzophénone aminosubstitué de formule (I) telle que définie ci-dessous permettait d'améliorer la stabilité et la coloration des compositions comprenant un agent autobronzant. Les colorations obtenues sont plus chromatiques, plus stables dans le temps et une bonne homogénéité.

La composition selon la présente invention comprend dans un support cosmétiquement acceptable au moins un dérivé de 2-hydroxybenzophénone aminosubstitué de formule (I) telle que définie ci-dessous et au moins un agent autobronzant.

La présente demande a encore pour objet l'utilisation de la composition selon l'invention à titre de composition destinée au bronzage ou au brunissement de la peau ; et un procédé cosmétique de bronzage ou brunissement de la peau tel qu'il consiste à appliquer sur la peau une quantité efficace d'une composition selon l'invention.

Enfin, la présente demande concerne encore l'utilisation d'au moins un dérivé de 2-hydroxybenzophénone aminosubstitué de formule (I) telle que définie ci-dessous dans des compositions pour le bronzage et/ou le brunissage artificiel de la peau contenant au moins un agent autobronzant en vue d'améliorer la coloration et/ou la stabilité dudit agent autobronzant.

Les compositions conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est extrêmement proche du bronzage naturel.

D'autres caractéristiques, aspects et avantages de la présente invention apparaîtront à la lecture de la description détaillée qui va suivre.

Les dérivés de 2-hydroxybenzophénone aminosubstitués conformes à l'invention répondent à la formule (I) suivante : 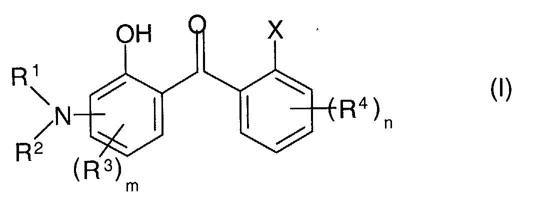 dans laquelle :
R¹ et R², identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀ ;
R¹ et R² peuvent également former avec l'atome d'azote avec lequel ils sont liés un cycle à 5 ou 6 chaînons ;
R³ et R⁴, identiques ou différents, désignent un radical alkyle en C₁-C₂₀, un radical alcènyle en C₂-C₁₀, un radical cycloalkyle en C₃-C₁₀, un radical cycloalcènyle en
C₃-C₁₀, un radical alcoxy en C1-C12, un radical (C₁-C₂₀)alcoxycarbonyle, un radical alkylamino en C₁-C₁₂, un radical dialkylamino en C₁-C₁₂, un radical aryle ou un hétéroaryle éventuellement substitué, un substituant hydrosolubilisant choisi parmi un groupe carboxylate, un groupe sulfonate ou un reste ammonium ;
X désigne un atome d'hydrogène, un groupe COOR⁵ ou CONR⁶R⁷;
R⁵, R⁶ et R⁷, identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀ , un groupe -(YO)ₒ-Z ou un groupe aryle ;
Y désigne -(CH₂)₂-, -(CH₂)₃- -(CH₂)₄-, -CH-CH₃-CH₂- ;
Z représente -CH₂-CH₃, -CH₂CH₂CH₃, -CH₂-CH₂-CH₂-CH₃, -CH(CH₃)-CH₃ ;
m est un entier variant de 0 à3 ;
n est un entier variant de 0 à 3 ;
o est un entier variant de 1 à 2.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : vinyle, n-propènyle, isopropènyle, 1-butènyle, 2-butènyle, 1-pentènyle, 2-pentènyle, 2-méthyl-1-butènyle, 2-méthyl-2-butènyle, 3-méthyl-1-butènyle, 1-hexènyle, 2-hexènyle, 1-heptènyle, 2-heptènyle, 1-octènyle, 2-octènyle.

Comme radicaux alcoxy en C₁-C₁₂, on peut citer : méthoxy, éthoxy, n-propoxy, n-butoxy, n-pentoxy, 1-méthylpropoxy, 3-méthylbutoxy, 2,2-diméthylpropoxy, 1-méthyl- 1-éthylpropoxy, octoxy, 2-méthylpropoxy, 1,1-diméthylpropoxy, hexoxy, heptoxy, 2-éthylhexoxy.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclopropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres peuvent être occupées par un hydrogène ou un radical alkyle en C₁-C₄.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Comme exemples de cycle à 5 ou 6 chaînons formé par les radicaux R¹ et R² avec l'atome d'azote, on peut citer en particulier pyrrolidine ou pipéridine.

Les groupes amino peuvent être fixés sur le noyau benzénique en position ortho, méta ou para par rapport au radical carbonyle et plus préférentiellement en para.

Une famille de composés de formule (I) préférentiels comprend ceux choisis parmi ceux de formule (la) suivante : 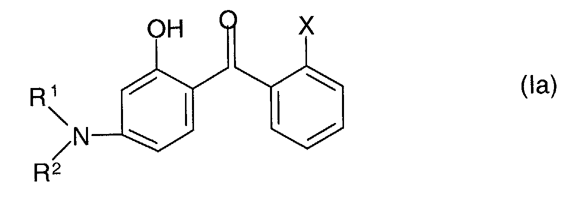 dans laquelle :
R¹ et R², identiques ou différents, désignent un atome d'hydrogène, un radical alkyle en C₁-C₁₂ ou forment avec l'atome d'azote avec lequel ils sont liés un cycle à 5 ou 6 chaînons ;
X désigne COOR⁵ ou CONR⁶R⁷ ;
R⁵ désigne un atome d'hydrogène, un radical alkyle en C₁-C₁₂, un radical cycloalkyle en C₃-C₆.
R⁶ et R⁷, identiques ou différents, désignent un atome d'hydrogène, un radical alkyle en C₁-C₁₂, un radical cycloalkyle en C₅-C₆.

Les composés de formule (la) plus particulièrement préférés sont ceux pour lesquels :
R¹ et R², identiques ou différents, désignent un radical alkyle en C₁-C₄ et plus particulèrement éthyle ;
R⁵ désigne un radical alkyle en C₃-C₈,
R⁶ et R⁷, identiques ou différents, désignent un radical alkyle en C₁-C₈,

Une autre famille de composés de formule (I) préférentiels comprend ceux choisis parmi ceux de formule (Ib) suivante : 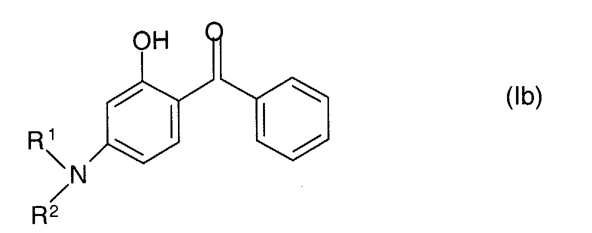 dans laquelle :
R¹ et R², identiques ou différents, désignent un radical alkyle en C₁-C₁₂ ou forment avec l'atome d'azote avec lequel ils sont liés un cycle à 5 ou 6 chaînons.

Parmi les composés de formule (Ib), on peut citer plus particulièrement :
- le (4-diéthylamino-2-hydroxyphényl)-phénylcétone.
- le (4-pyrrolidino-2-hydroxyphényl)-phénylcétone.

Une famille de composés de formule (I) plus particulièrement préférés comprend ceux choisis parmi ceux de formule (Ic) suivante : 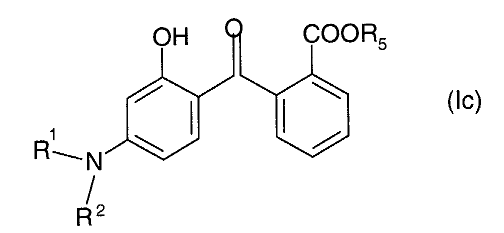 dans laquelle :
R¹ et R², identiques ou différents, désignent un atome d'hydrogène, un radical alkyle en C₁-C₈ ou forment avec l'atome d'azote avec lequel ils sont liés un cycle à 5 ou 6 chaînons ;
R⁵ désigne un atome d'hydrogène, un radical alkyle en C₁-C₁₂, un radical cycloalkyle en C₃-C₆.

Parmi les composés de formule (lc), on peut citer :
- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

Un composé de formule (I) tout particulièrement préféré est le 2-(4-diéthylamino-2-hydroxybenzoyl)-benzoate de n-hexyle.

Les composés de formule (I) tels que définis ci-dessus sont connus en eux-mêmes et leurs structures et leurs synthèses sont décrites dans les demandes de brevet EP-A-1046391 et DE100 12 408 (faisant partie intégrante du contenu de la description).

Les dérivés de 2-hydroxybenzophénone aminosubstitués conformes à l'invention sont présents de préférence dans la composition de l'invention dans des proportions allant de 0,1 à 15 % en poids et plus préférentiellement de 1 à 10% en poids et plus particulièrement de 2 à 8% en poids par rapport au poids total de la composition.

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazoline-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Ces agents autobronzants peuvent être associés à au moins un colorant direct synthétique ou naturel et/ou au moins un dérivé indolique comme ceux décrits dans les brevets EP 425 324 et EP 456 545.

Ces agents autobronzants peuvent également être associés à d'autres agents de coloration de la peau synthétiques ou naturels.

Au sens de la présente invention, on entendra par « agent de coloration de la peau », tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage.

Les agents de coloration additionnels peuvent également être choisis par exemple parmi les extraits végétaux comme par exemple les extraits de bois rouges « insolubles » du genre Pterocarpus et du genre Baphia comme le Pterocarpus santalinus, le Pterocarpus osun, le Pterocarpus soyauxii, le Pterocarpus erinaceus, le Pterocarpus indicus ou encore le Baphia nitida comme ceux décrits dans la demande de brevet EP 971 683.

Les agents de coloration peuvent également être des nanopigments d'oxyde de fer dont la taille moyenne des particules élémentaires est inférieure à 100nm tels que ceux décrits dans la demande de brevet EP 966 953.

Les agents autobronzants sont généralement présents dans les compositions selon l'invention dans des proportions allant de 0,1 à 10% en poids par rapport au poids total de la composition, et de préférence de 0,2 à 8% en poids par rapport au poids total de la composition.

Les compositions autobronzantes conformes à l'invention peuvent se présenter sous forme de crèmes, de laits, de gels, de gel-crèmes, d'émulsions huile-dans-eau, de dispersions vésiculaires, de lotions fluides, en particulier de lotions fluides vaporisables ou tout autre forme généralement utilisée en cosmétique, en particulier celles convenant habituellement aux compositions cosmétiques autobronzantes.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les antagonistes de substance P, les anti-inflammatoires, les parfums, les conservateurs, les tensioactifs, les charges, les polymères autres que ceux de l'invention, les propulseurs, les agents alcanisants ou acidifiants, les colorants ou tout autre ingrédient habituellement utilisé dans le domaine cosmétique et/ou dermatologique, en particulier pour la fabrication de compositions autobronzantes sous forme d'émulsions.

Les corps gras peuvent être constitués par une huile ou une cire ou leurs mélanges. Par « huile », on entend un composé liquide à température ambiante. Par « cire », on entend un composé solide ou substantiellement solide à température ambiante, et dont le point de fusion est généralement supérieur à 35°C.

Comme huiles, on peut citer les huiles minérales (paraffine) ; végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société Finetex, le palmitate d'octyle, le lanolate d'isopropyle, les triglycérides dont ceux des acides caprique/caprilyque, les esters et les éthers gras oxyéthylénés ou oxypropylénés ; siliconées (cyclométhicone, polydiméthyl-siloxanes ou PDMS) ou fluorées; les polyalkylènes et leurs mélanges.

Comme composés cireux, on peut citer la paraffine, la cire de carnauba, la cire d'abeille, l'huile de ricin hydrogénée.

Parmi les solvants organiques, on peut citer les alcools et polyols inférieurs ayant au plus 8 atomes de carbone.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthylcellulose.

Les compositions conformes à l'invention peuvent comporter en plus au moins un agent photoprotecteur organique et/ou au moins un agent photoprotecteur inorganique actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés cinnamiques ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone autres que ceux de formule (I) ; les dérivés de β,β'-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 .; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586.

Comme exemples d'agents photoprotecteurs organiques actifs dans l'UV-A et/ou l'UV-B, on peut citer désignés ci-dessus sous leur nom INCI :

### Dérivés de l'acide para-aminobenzoïque :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF.

### Dérivés salicyliques :

Homosalate vendu sous le nom « EUSOLEX HMS » par RONA/EM INDUSTRIES,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate vendu sous le nom « NEO HELIOPAN TS » par HAARMANN et REIMER.

### Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane.

### Dérivés cinnamiques:

Ethylhexyl Methoxycinnamate vendu notamment sous le nom commercial « PARSOL MCX » par HOFFMANN LAROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
Glyceryl Ethylhexanoate Dimethoxycinnamate.

### Dérivés de β-β'-diphénylacrylate :

Octocrylene vendu notamment sous le nom commercial « UVINUL N539 » par BASF,
Etocrylene vendu notamment sous le nom commercial « UVINUL N35 » par BASF.

### Dérivés de la benzophénone :

Benzophenone-1 vendu sous le nom commercial « UVINUL 400 » par BASF,
Benzophenone-2 vendu sous le nom commercial « UVINUL D50 » par BASF,
Benzophenone-3 ou Oxybenzone vendu sous le nom commercial « UVINUL M40 » par BASF,
Benzophenone-4 vendu sous le nom commercial « UVINUL MS40 » par BASF,
Benzophenone-5,
Benzophenone-6 vendu sous le nom commercial « HELISORB 11 » par NORQUAY,
Benzophenone-8 vendu sous le nom commercial « SPECTRA-SORB UV-24 » par AMERICAN CYANAMID,
Benzophenone-9 vendu sous le nom commercial « UNIVUL DS-49 » par BASF, Benzophenone-12.

### Dérivés du benzylidène camphre :

3-Benzylidene camphor fabriqué sous le nom « MEXORYL SD » par CHIMEX,
4-Methylbenzylidene camphor vendu sous le nom « EUSOLEX 6300 » par MERCK,
Benzylidene Camphor Sulfonic Acid fabriqué sous le nom « MEXORYL SO » par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX »par CHIMEX,
Polyacrylamidomethyl Benzylidene Camphor fabriqué sous le nom « MEXORYL SW » par CHIMEX.

### Dérivés de benzimidazole :

Phenylbenzimidazole Sulfonic Acid vendu notamment sous le nom commercial « EUSOLEX 232 » par MERCK,
Benzimidazilate vendu sous le nom commercial « NEO HELIOPAN AP » par HAARMANN et REIMER.

### Dérivés de triazine :

Anisotriazine vendu sous le nom commercial « TINOSORB S » par CIBA SPECIALITY CHEMICALS,
Ethylhexyl triazone vendu notamment sous le nom commercial « UVINUL T150 » par BASF,
Diethylhexyl Butamido Triazone vendu sous le nom commercial « UVASORB HEB » par SIGMA 3V,
La 2,4,6-tris-(4'-amino benzalmalonate de diisobutyle)-s-triazine.

### Dérivés de benzotriazole :

Drometrizole Trisiloxane vendu sous le nom « SILATRIZOLE » par RHODIA CHIMIE,
Methylène bis-Benzotriazolyl Tetramethylbutylphénol vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALITY CHEMICALS.

### Dérivés anthraniliques:

Menthyl anthranilate vendu sous le nom commercial « NEO HELIOPAN MA » par HAARMANN et REIMER.

### Dérivés d'imidazolines :

Ethylhexyl Dimethoxybenzylidene Dioxoimidazoline Propionate.

### Dérivés de benzalmalonate :

Polyorganosiloxane à fonctions benzalmalonate vendu sous la dénomination commerciale « PARSOL SLX » par HOFFMANN LAROCHE

### Dérivés de 4,4-diarylbutadiene

1,1-dicarboxy (2,2'-diméthyl-propyl)-4,4-diphénylbutadiène.
et leurs mélanges.

Les agents photoprotecteurs organiques plus particulièrement préférés sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic Acid,
Benzophenone-3,
Benzophenone-4,
Benzophenone-5,
4-Methylbenzylidene Camphor,
Benzimidazilate,
Anisotriazine,
Ethylhexyl triazone,
Diethylhexyl Butamido Triazone,
Methylene bis-Benzotriazolyl Tetramethylbutylphenol,
Drometrizole Trisiloxane,
2,4,6-tris-(4'-amino benzalmalonate de diisobutyle)-s-triazine,
1,1-dicarboxy (2,2'-diméthyl-propyl)-4,4-diphénylbutadiène
et leurs mélanges.

Les agents photoprotecteurs inorganiques sont choisis parmi les pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium et leurs mélanges. Des agents d'enrobage classiques sont par ailleurs l'alumine et/ou le stéarate d'aluminium. de tels nanopigments d'oxydes métalliques, enrobés ou non enrobés, sont en particulier décrits dans les demandes de brevets EP 518 772 et EP 518 773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans-huile.

Lorsqu'il s'agit d'une émulsion, la phase aqueuse de celle-ci peut comprendre une dispersion vésiculaire non ionique préparée selon les procédés connus (Bangham, Standish and Watkins J. Mol. Biol. 13, 238 (1965), FR 2 315 991 et FR 2 416 008).

L'invention concerne également un procédé de traitement cosmétique pour bronzer et/ou brunir artificiellement la peau, caractérisé par le fait qu'il consiste à appliquer sur celle-ci une quantité efficace d'une composition cosmétique telle que définie ci-dessus.

L'invention concerne également l'utilisation d'un dérivé de 2-hydroxybenzophénone aminosubstitué de formule (I) tel que défini précédemment dans le but d'améliorer la coloration et/ou la stabilité d'un agent autobronzant tels que ceux définis ci-dessus contenu dans une composition cosmétique destinée au bronzage et/ou brunissage artificiels de la peau.

Des exemples concrets, mais nullement limitatifs, illustrant l'invention, vont maintenant être donnés.

| **Exemple 1** | |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1 g |
| Benzoate d'alcools en C₁₂-C₁₅ (WITCONOL TN -WITCO) | 12g |
| 2-(4-diethylamino-2-hydroxybenzoyl)-benzoic acid hexylester | 3g |
| Glycérine | 10g |
| Dihydroxyacetone | 5g |
| Conservateurs | qs |
| Eau déminéralisée qsp | 100 g |

| **Exemple 2** | |
|---|---|
| Gomme de xanthane | 1g |
| Copolymère acide acrylique/acrylate d'alkyle (C10/C30 )réticulé (Pemulen TR2 - GOODRICH) | 0.4g |
| Triéthanolamine | 0.4g |
| Benzoate d'alcools en C₁₂-C₁₅ (WITCONOL TN -WITCO) | 10g |
| 2-(4-diethylamino-2-hydroxybenzoyl)-benzoate de n-hexyle | 1.5g |
| Glycérine | 5g |
| Dihydroxyacétone | 5g |
| Conservateurs | qs |
| Eau distillée qsp | 100 g |

## Revendications

1. Composition cosmétique et/ou dermatologique, **caractérisée par le fait qu'**elle contient dans un support cosmétiquement acceptable :
(i) au moins un agent autobronzant,
(ii) au moins un dérivé de 2-hydroxybenzophénone amino-substitué de formule (I) suivante : 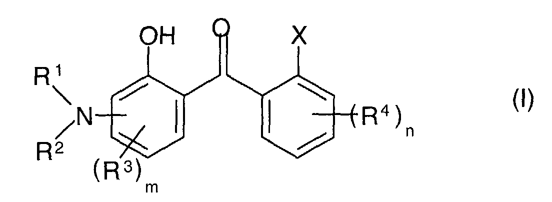
dans laquelle :
R¹ et R², identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀ ;
R¹ et R² peuvent également former avec l'atome d'azote avec lequel ils sont liés un cycle à 5 ou 6 chaînons ;
R³ et R⁴, identiques ou différents, désignent un radical alkyle en C₁-C₂₀, un radical alcènyle en C₂-C₁₀, un radical cycloalkyle en C₃-C₁₀, un radical cycloalcènyle en C₃-C₁₀, un radical alcoxy en C1-C12, un radical (C₁-C₂₀)alcoxycarbonyle, un radical alkylamino en C₁-C₁₂, un radical dialkylamino en C₁-C₁₂, un radical aryle ou un hétéroaryle éventuellement substitué, un substituant hydrosolubilisant choisi parmi un groupe carboxylate, un groupe sulfonate ou un reste ammonium ;
X désigne un atome d'hydrogène, un groupe COOR⁵ ou CONR⁶R⁷;
R⁵, R⁶ et R⁷, identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀, un groupe -(YO)ₒ-Z ou un groupe aryle ;
Y désigne -(CH₂)₂-, -(CH₂)₃- -(CH₂)₄-, -CH-CH₃-CH₂- ;
Z représente -CH₂-CH₃, -CH₂CH₂CH₃, -CH₂-CH₂-CH₂-CH₃, -CH(CH₃)-CH₃;
m est un entier variant de 0 à3 ;
n est un entier variant de 0 à 3 ;
o est un entier variant de 1 à 2 .

2. Composition selon la revendication 1 où le ou les composés de formule (I) sont choisis parmi ceux de formule (la) suivante : 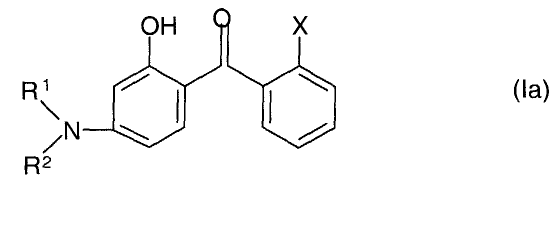 dans laquelle :
R¹ et R², identiques ou différents, désignent un atome d'hydrogène, un radical alkyle en C₁-C₁₂ ou forment avec l'atome d'azote avec lequel ils sont liés un cycle à 5 ou 6 chaînons ;
X désigne COOR⁵ ou CONR⁶R⁷;
R⁵ désigne un atome d'hydrogène, un radical alkyle en C₁-C₁₂, un radical cycloalkyle en C₃-C₆.
R⁶ et R⁷, identiques ou différents, désignent un atome d'hydrogène, un radical alkyle en C₁-C₁₂, un radical cycloalkyle en C₅-C₆.

3. Composition selon la revendication 2, où les composés de formule (la) sont ceux pour lesquels :
R¹ et R², identiques ou différents, désignent un radical alkyle en C₁-C₄ et plus particulièrement éthyle ;
R⁵ désigne un radical alkyle en C₃-C₈,
R⁶ et R⁷ identiques ou différents, désignent un radical alkyle en C₁-C₈,

4. Composition selon la revendication 1 où le ou les composés de formule (I) sont choisis parmi ceux de formule (Ib) suivante : 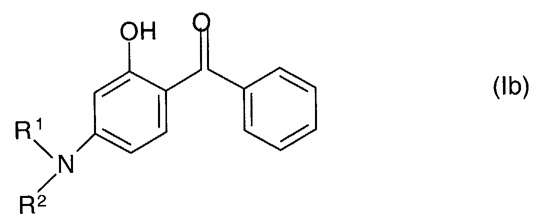 dans laquelle :
R¹ et R², identiques ou différents, désignent un radical alkyle en C₁-C₁₂ ou forment avec l'atome d'azote avec lequel ils sont liés un cycle à 5 ou 6 chaînons.

5. Composition selon la revendication 4, où le composé de formule (Ib) est choisi parmi :
- le (4-diéthylamino-2-hydroxyphényl)-phénylcetone.
- le (4-pyrrolidino-2-hydroxyphényl)-phénylcétone.

6. Composition selon la revendication 1 où le ou les composés de formule (I) sont choisis parmi ceux de formule (Ic) suivante : 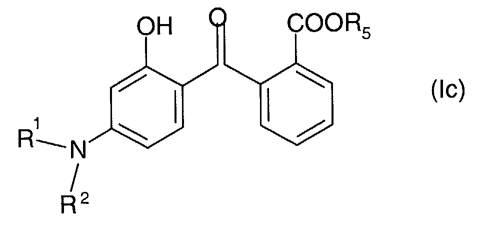 dans laquelle :
R¹ et R², identiques ou différents, désignent un atome d'hydrogène, un radical alkyle en C₁-C₈ ou forment avec l'atome d'azote avec lequel ils sont liés un cycle à 5 ou 6 chaînons ;
R⁵ désigne un atome d'hydrogène, un radical alkyle en C₁-C₁₂, un radical cycloalkyle en C₃-C₆.

7. Composition selon la revendication 6 où le composé de formule (lc) est choisi parmi:
- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

8. Composition selon la revendication 7 où le composé de formule (lc) est le 2-(4-diéthylamino-2-hydroxybenzoyl)-benzoate de n-hexyle

9. Composition selon l'une quelconque des revendications 1 à 8, dans laquelle le ou les dérivés de 2-hydroxybenzophénone aminosubstitués de formule (I) sont présents dans des proportions allant de 0,1 % à 15 % en poids

10. Composition selon la revendication 9, dans laquelle le ou les dérivés de 2-hydroxybenzophénone aminosubstitués de formule (I) sont présents dans des proportions allant de 1 à 10% en poids

11. Composition selon la revendication 10, dans laquelle le ou les dérivés de 2-hydroxybenzophénone aminosubstitués de formule (I) sont présents dans des proportions allant de 2 à 8% en poids par rapport au poids total de la composition.

12. Composition selon l'une des revendications 1 à 11, telle que l'agent autobronzant est un composé mono ou polycarbonylé.

13. Composition selon la revendication 12, telle que l'agent autobronzant est choisi dans le groupe formé par l'isatine, l'alloxane, la ninhydrine, le glycéraldéhyde, l'aldéhyde mésotartrique, la glutaraldéhyde, l'érythrulose, les dérivés de pyrazoline -4,5-dione, la dihydroxyacétone (DHA), les dérivés de 4,4-dihydroxypyrazoline-5-one.

14. Composition selon la revendication 13, telle que l'agent autobronzant est la DHA.

15. Composition selon l'une quelconque des revendications 1 à 14, telle que la concentration en agent autobronzant varie de 0,1 à 10% en poids, par rapport au poids total de la composition.

16. Composition selon l'une des revendications 1 à 15, **caractérisée par le fait qu'**elle comporte au moins un colorant direct, synthétique ou naturel, et/ou au moins un dérivé indolique.

17. Composition selon l'une des revendications 1 à 15, telle qu'elle comprend un agent de coloration additionnel choisi parmi les extraits de bois rouges "insolubles" du genre Pterocarpus et du genre Baphia .

18. Composition selon l'une des revendications 1 à 15, telle qu'elle comprend un agent de coloration additionnel choisi parmi les nanopigments d'oxyde de fer dont la taille moyenne des particules élémentaires est inférieure à 100 nm.

19. Composition selon l'une quelconque des revendications 1 à 18, **caractérisée par le fait qu'**elle comporte au moins un adjuvant cosmétique choisi parmi les corps gras, les solvants organiques, les émulsionnants, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les agents répulsifs contre les insectes, les antagonistes de substance P, les anti-inflammatoires, les parfums, les conservateurs, les tensioactifs, les charges, les polymères, les propulseurs, les agents alcalinisants ou acidifiants.

20. Composition selon l'une quelconque des revendications 1 à 19, **caractérisée par le fait qu'**elle comporte en plus au moins un agent photoprotecteur organique et/ou au moins un agent photoprotecteur inorganique actifs dans l'UVA et/ou dans l'UVB.

21. Composition selon la revendication 20, telle que l'agent photoprotecteur organique est choisi parmi les dérivés 1,3,5-triazine, dérivés du dibenzoylméthane, les dérivés cinnamiques, les anthranilates ; les dérivés salicyliques, les dérivés du camphre ; les dérivés de la benzophénone autres que ceux de formule (I) ; les dérivés de β,β-diphénylacrylate, les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-hydroxyphényl benzotriazole ; les polymères filtres et silicones filtres; les dimères dérivés d'α-alkylstyrène et les 4,4-diarylbutadiènes et leurs mélanges.

22. Composition selon la revendication 21, telle que l'agent photoprotecteur organique est choisi parmi :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic,
Benzophenone-3,
Benzophenone-4,
Benzophenone-5,
4-Methylbenzylidene Camphor,
Benzimidazilate,
Anisotriazine,
Ethylhexyl triazone,
Diethylhexyl Butamido Triazone,
Methylene bis-Benzotriazolyl Tetramethylbutylphenol,
Drometrizole Trisiloxane,
la 2,4,6-tris-(4'-amino benzalmalonate de diisobutyle)-s-triazine
1,1-dicarboxy (2,2'-diméthyl-propyl)-4,4-diphénylbutadiène
et leurs mélanges.

23. Composition selon la revendication 20, telle que l'agent photoprotecteur inorganique est choisi parmi les pigments ou les nanopigments d'oxydes métalliques enrobés ou non.

24. Composition selon la revendication 23, telle que l'agent photoprotecteur organique est choisi parmi les nanopigments enrobés ou non, d'oxyde de titane, de fer, de zinc, de zirconium ou de cérium et leurs mélanges.

25. Composition selon l'une quelconque des revendications 20 à 24 telle que l'agent photoprotecteur est présent dans les compositions dans des proportions allant de 0,1 à 20% en poids par rapport au poids total de la composition, et de préférence allant de 0,2 à 15% en poids par rapport au poids total de la composition.

26. Composition selon l'une des revendications précédentes, telle qu'elle se présente sous la forme d'une dispersion vésiculaire non ionique, d'une émulsion, en particulier d'une émulsion de type eau-dans huile, de type huile-dans-eau, d'une crème, ou d'une émulsion triple (E/H/E ou H/E/H), d'un lait, d'un gel, d'un gel crème, d'une suspension, d'une dispersion, d'une mousse ou d'un spray.

27. Utilisation d'une composition selon la revendication 26 à titre de composition cosmétique destinée au bronzage ou au brunissement de la peau.

28. Procédé cosmétique de bronzage ou brunissement de la peau **caractérisé en ce qu'**il consiste à appliquer sur la peau une quantité efficace d'une composition cosmétique selon l'une des revendications 1 à 26.

29. Utilisation d'au moins un dérivé de 2-hydroxybenzophénone aminosubstitué tel que défini dans l'une quelconque des revendications 1 à 8 dans une composition pour le bronzage et/ou le brunissage artificiels de la peau contenant au moins un agent autobronzant dans le but d'améliorer la coloration et/ou la stabilité dudit agent autobronzant.
